# EUROPEAN PATENT APPLICATION

(11) **EP 4 502 183 A1**
(43) Date of publication of application: **05.02.2025**
(21) Application number: 23382807.8
(22) Date of filing: 02.08.2023
(51) Int. Cl.: C12Q 1/6886

(54) **METHOD FOR DETERMINING THE HOMOLOGOUS RECOMBINATION DEFICIENCY STATUS OF A TUMOR AND FOR PREDICTING THE RESPONSE OF A CANCER TO A THERAPY**

(71) Applicant: Fundació Privada Institut d'Investigació Oncològica de Vall Hebron, 08035 Barcelona (ES)
(72) Inventor: VIVANCOS PRELLEZO, Ana, E-08035 Barcelona (ES); ROMERO LOZANO, Paula, E-08035 Barcelona (ES); VILA CASADESUS, María, E-08035 Barcelona (ES); CASTILLO ANDREO, Ester, E-08035 Barcelona (ES)
(74) Representative: ABG Intellectual Property Law, S.L.

(57) **Abstract**

The present invention is directed to a method for determining the HRD status of a tumour. More particularly, the invention is directed to a method for predicting the response of a cancer patient to an antitumor treatment.

## Description

### FIELD OF THE INVENTION

The invention relates to the field of precision medicine and, in particular, to methods for predicting the response of a cancer patient to an anticancer therapy as well as for the determination of the homologous recombination deficiency (HRD) status of a tumor.

### BACKGROUND ART

Cancer is one of the most important diseases in the world. This disease is a leading cause of death worldwide, accounting for nearly 10 million deaths in 2020. The incidence of cancer rises dramatically with age, most likely due to a build-up of risks for specific cancers that increase with age. The overall risk accumulation is combined with the tendency for cellular repair mechanisms, such as homologous recombination, to be less effective as a person grows older.

Homologous recombination deficiency is a tumor characteristic that is defined by the inability to accurately repairs double strand breaks in DNA via homologous recombination. The majority of homologous recombination deficient (HRD) tumors will occur in patients with germline BRCA1 and BRCA2 mutations. However, there also are patients with germline mutations in other HR pathway genes and patients who do not carry an inherited germline mutation but have tumors with sporadic HRD mutations. Accurate monitoring of HRD is clinically important due to its role in chemotherapy, targeted therapy and immunotherapy. Platinum salts are one of the most important chemotherapeutic drugs with a broad anticancer spectrum that introduces DNA double stranded breaks and interstrand crosslinks, being effective in HRD tumors. HR-deficient cells are also sensitive to poly-ADP ribose polymerase inhibitors (PARPi), whose action is based on inhibiting the single-stranded breaks repairing and the accumulation of fatal double-stranded breaks. HRD is a potential biomarker for platinum salts and PARPi in many cancers but especially in breast and ovarian. Moreover, patients who have exquisite response to neoadjuvant platinum-based chemotherapy may be more likely to be HRD and hence benefit from PARPi treatment. Clinically, HR status in ovarian cancer is a biomarker of PARPi response and required for proper patient management. Current methods for detecting homologous recombination repair defects include, among others, detecting mutations or LOH (Loss of Heterozygosity) in BRCA1/2 and other genes involved in homologous recombination in tissue samples and blood samples, detection of mutations in HRD-related genes or genome-wide SNP-based analysis to determine loss of heterozygosity, telomere imbalance, and large fragment migration.

However, there is still a need for the identification and development of effective biomarkers of the homologous recombination (HR) status of tumors.

### SUMMARY OF THE INVENTION

The object of the present invention is to overcome the deficiencies of the prior art and to provide a method for determining the HRD status of cancer either from a tumor sample or a liquid biopsy. The inventive method has been developed based on the hypothesis that CNAs in certain genomic regions (regardless of being gained or lost) recurrently in HRD positive tumors (defined as by having an HRD-LOH>42 score).

The authors of the present invention have developed, by way of extensive experimentation, a method for the determination of the HRD status of a cancer present in a patient which comprising determining in sample from the patient the presence of a series of copy number alterations (CNA) in certain genomic regions.

Since the presence of HRD correlates with sensitivity to certain anticancer treatments, the method developed by the inventors is also useful in predicting the response of the patient to said antitumor treatments.

Thus, in a first aspect, the present invention relates to an in vitro method for determining the HRD status of a tumor comprising: determining in a sample of said tumor the presence of at least one CNA in a genomic region selected from the group consisting of the genomic regions as shown in Table 1 wherein the presence of said at least one CNA is correlated with a HRD status.

In a second aspect, the invention provides an in vitro method for predicting tumor deficiency in the DNA homologous recombination (HR) pathway in a patient suffering from cancer, comprising determining in the genetic material of a sample from said patient the presence of CNA in at least one genomic region selected from the group consisting of the genomic regions as shown in Table 1, wherein the presence of said at least one CNA indicates that the tumor in the patient has a HR-deficient status.

In a third aspect the present invention relates to a method for predicting the response of a cancer patient to an antitumor treatment comprising: determining in the genetic material of a sample from said patient the presence of at least one CNA in a genomic region selected from the group consisting of the genomic regions as shown in Table 1, wherein the presence of said at least one CNA indicates that the cancer is response to an antitumor treatment selected from the group consisting of a chemotherapy, targeted therapy or immunotherapy.

In yet another aspect, the invention relates to a method for the treatment of a cancer in a patient which comprises predicting the response of the cancer to an antitumor treatment by the method according to the invention and treating the patient with an antitumor treatment selected from the group consisting of a chemotherapy, targeted therapy or immunotherapy is the patient is identified as being responsive to the tumor treatment.

### BRIEF DESCRIPTION OF THE FIGURES

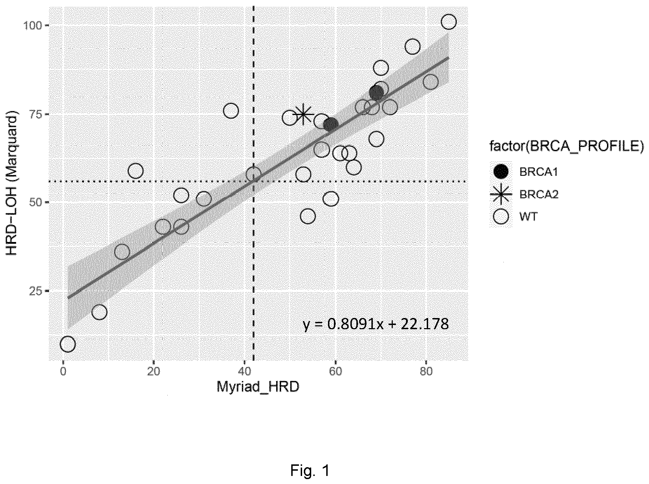

**Figure 1****:** Myriad score correlation with HRD-LOH for primary ovarian and breast cancer. Scatter plot showing the correlation between Myriad score (x axis) and HRD-LOH (Marquard) score (y axis) for primary ovarian and breast cancer. Dotted lines indicate the cut-off values applied in Myriad (≥ 42) and in the HRD-LOH (Marquard) (≥ 56). The line of best fit from a robust linear regression is shown in bold black with the line of best fit equation on the bottom right of each plot. Confidence boundary area shadowed in gray. Samples with a BRCA 1 mutation in a LOH region are represented as filled black dots (BRCA1), BRCA 2 mutation presenting LOH represented as asterisks (BRCA2), wild type samples represented as circles (WT).

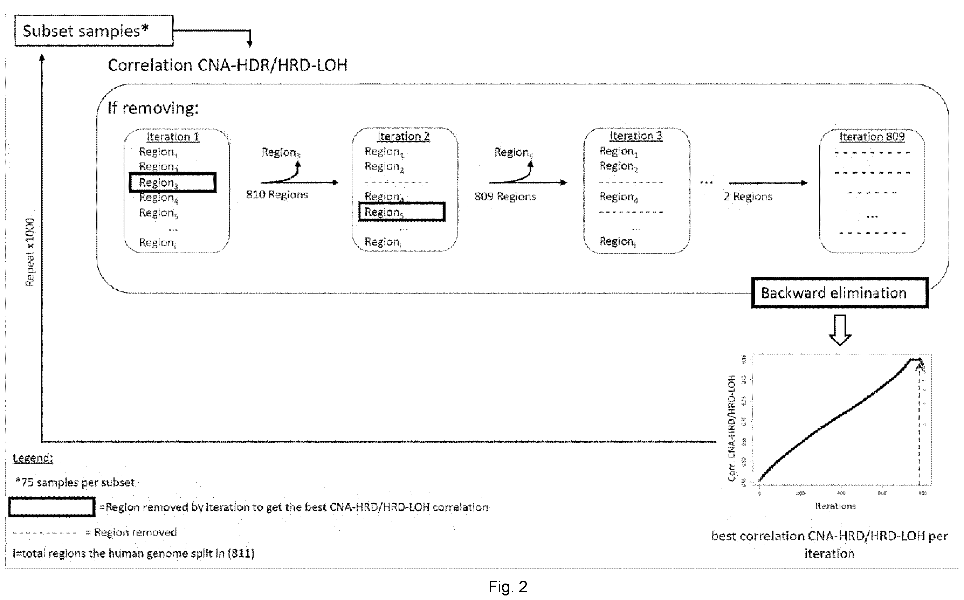

**Figure 2****.** Methodology of the selection model
   Backward elimination method used to select the regions that compounds the CNA-HRD score that best correlates with the HRD-LOH Score. CNA-HRD/HRD-LOH correlation was determined removing each single region contained in each iteration and encircled the region removed per iteration to achieve the best CNA-HRD/HRD-LOH correlation. Plot shows CNA-HRD/HRD-LOH correlation for all the iterations, arrow indicates best iteration set of the subset of samples to correlate CNA-HRD/HRD-LOH (iteration 779).

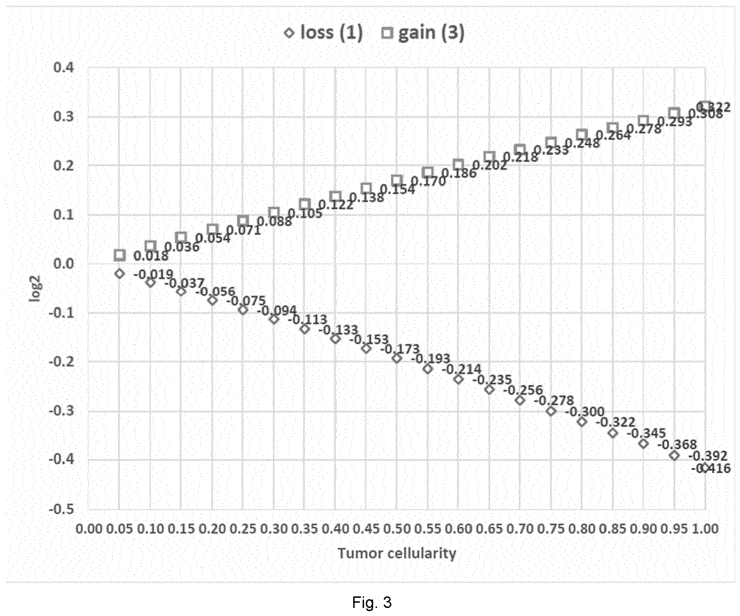

**Figure 3****.** Graphical representation of the log2 cut-off value selected by tumor cellularity and CNA (gain or loss). Log2 values for copy number gain is represented as squares and loss as diamonds.

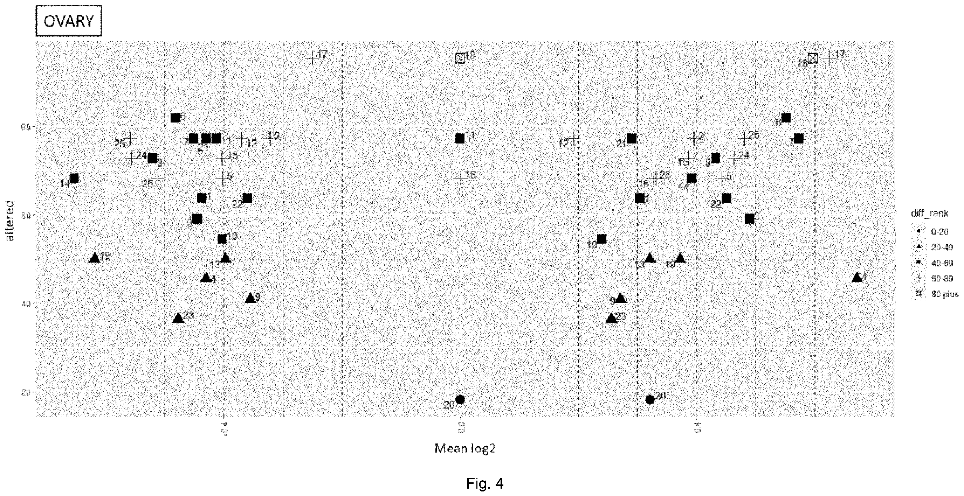

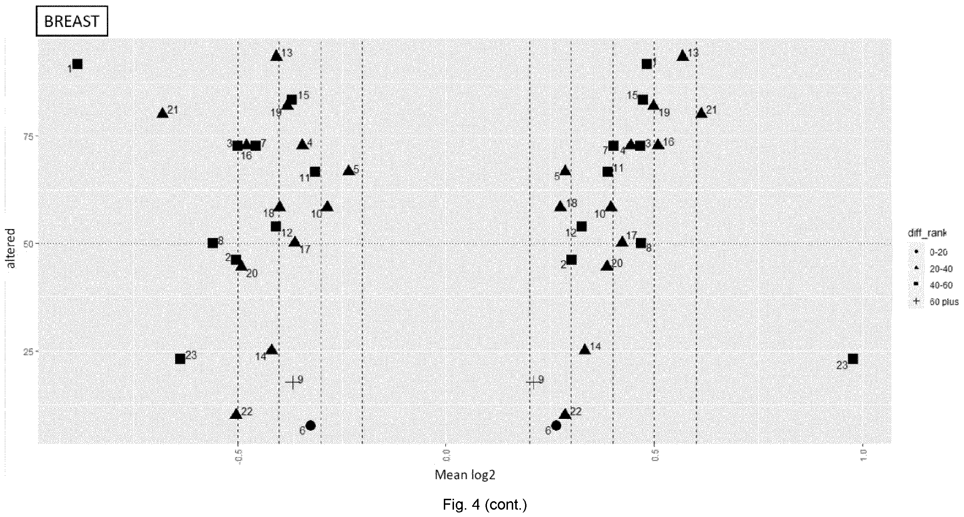

**Figure 4****.** Scatter plot showing the distribution of the selected regions for primary ovarian (top) and breast (bottom) tumor samples based on the mean log2 of each region (x axis) and its percentage altered (y axis). The regions are shaped based on the alteration difference between HRD-LOH positive and HRD-LOH negative.

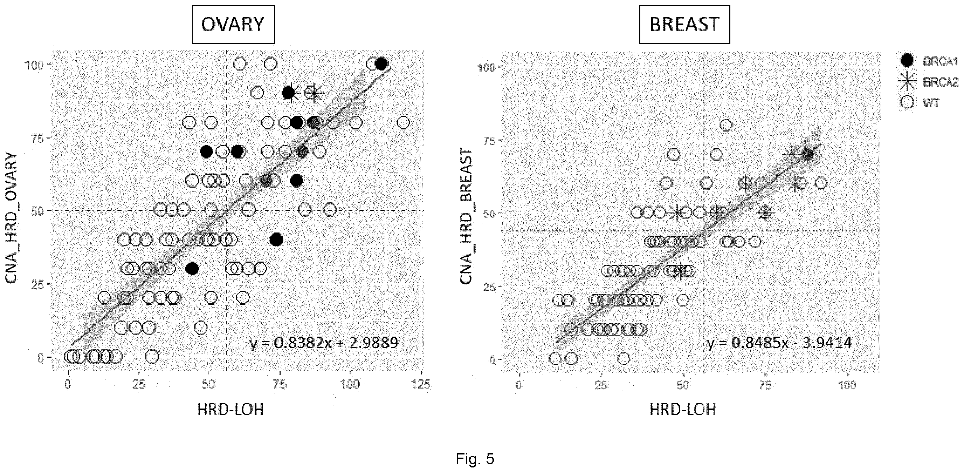

**Figure 5****.** CNA-HRD correlation with HRD-LOH for primary ovarian and breast cancer using the 10 regions selected respectively. Scatter plot showing the correlation between HRD-LOH score (x axis) and CNA-HRD score (y axis) for ovarian (left side) and breast (right side) cancer samples. Dotted lines indicate the cut-off values applied in the HRD-LOH (≥ 56) and in the CNA-HRD (≥ 50 & ≥ 44) for the ovarian and breast cancer, respectively. The line of best fit from a robust linear regression is shown in bold black. Confidence boundary area shadowed in gray. Samples with a BRCA 1 mutation in a LOH region are represented as filled black dots (BRCA1), BRCA 2 mutation presenting LOH represented as asterisks (BRCA2), wild type samples represented as circles (WT).

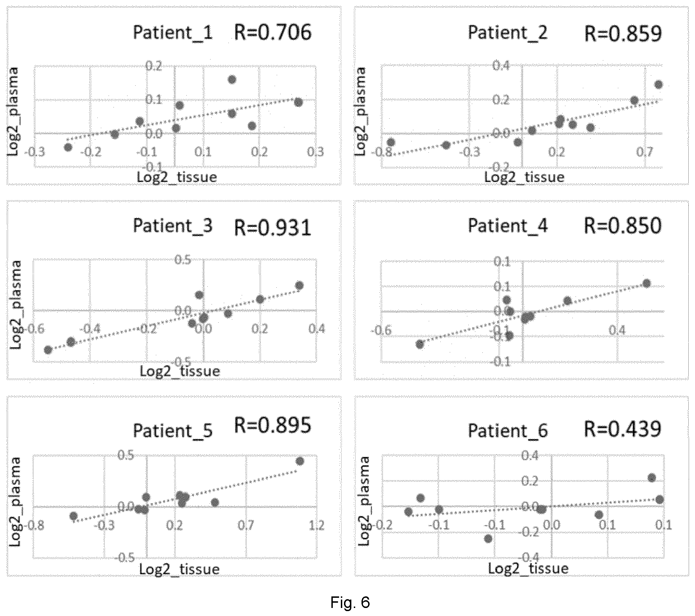

**Figure 6****.** Log2 values correlation between the 10 regions selected for CNA-HRD breast for tissue and plasma.

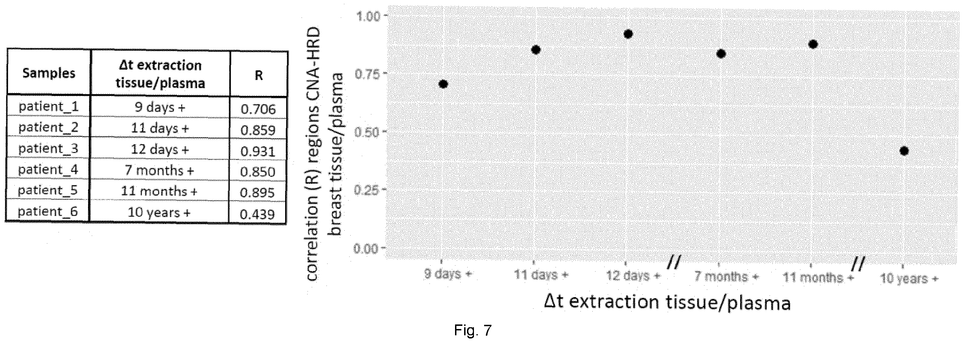

**Figure 7****.** Correlation between tissue and plasma when using the 10 regions selected for CNA-HRD breast over time. Table on the left shows the time difference between tissue and plasma extraction (1-6), along with the Log2 values correlation between the regions selected for CNA-HRD breast for tissue and plasma. On the right, scatter plot showing correlation between partner samples when using CNA-HRD score breast (Y axis) over time (X axis).

### DETAILED DESCRIPTION OF THE INVENTION

The present invention relates to the provision of a method for determining homologous recombination deficiency status.

Unless otherwise defined, all technical terms used herein have the same meaning as commonly understood by one of ordinary skill in the art to which this invention belongs. All the embodiments and definitions disclosed in the context of one aspect of the invention are also applicable to the other aspects of the invention.

### Method for determining HR status of a tumor

The authors of the present invention have discovered that the presence of a CNA or the combination of CNAs in one or more genomic regions in the genome in a tumor correlate with the HR status of a tumor. In this way, the presence of the CNA in one or more genomic regions acts as a surrogate marker for the presence of HR deficiency or HRD,

Accordingly, in a first aspect, the invention relates to *in vitro* method for determining the homologous recombination (HR) status of a tumor, comprising determination in a biological sample of said tumor the presence of a copy number alteration (CNA) in at least one genomic region, wherein the genomic region is selected from the group consisting of the genomic region as shown in Table 1 wherein the presence of said at least one CNA in at least one genomic region is correlated with a HR deficient status.

The term "homologous recombination deficiency (HRD)", as used herein, refers to a phenotype that is characterized by the inability of a cell to effectively repair DNA double-strand breaks using the homologous recombination repair (HRR) pathway. It is a cause of chromosome instability and a common characteristic of many tumors. It is clinically important due to its role in chemotherapy, and targeted therapy and immunotherapy.

The term "tumor", as used herein, refers to a mass formed by the abnormal and excessive growth of tissue.. Included in this definition are benign and malignant cancers and includes early stage tumor as well as tumors that are invasive and which are classified stage 0, 1, or 2 cancer. Examples of a tumors include those that can be found in the cancer types that include, but are not limited to, a lung cancer (e.g., a non-small cell lung cancer (NSCLC)), a kidney cancer (e.g., a kidney urothelial carcinoma), a bladder cancer (e.g., a bladder urothelial (transitional cell) carcinoma), a breast cancer, a colorectal cancer (e.g., a colon adenocarcinoma), an ovarian cancer, a pancreatic cancer, a gastric carcinoma, an esophageal cancer, a mesothelioma, a melanoma (e.g., a skin melanoma), a head and neck cancer (e.g., a head and neck squamous cell carcinoma (HNSCC)), a thyroid cancer, a sarcoma (e.g., a soft-tissue sarcoma, a fibrosarcoma, a myxosarcoma, a liposarcoma, an osteogenic sarcoma, an osteosarcoma, a chondrosarcoma, an angiosarcoma, an endotheliosarcoma, a lymphangiosarcoma, a lymphangioendotheliosarcoma, a leiomyosarcoma, or a rhabdomyosarcoma), a prostate cancer, a glioblastoma, a cervical cancer, a thymic carcinoma, a leukemia (e.g., an acute lymphocytic leukemia (ALL), an acute myelocytic leukemia (AML), a chronic myelocytic leukemia (CML), a chronic eosinophilic leukemia, or a chronic lymphocytic leukemia (CLL)), a lymphoma (e.g., a Hodgkin lymphoma or a non-Hodgkin lymphoma (NHL)), a myeloma (e.g., a multiple myeloma (MM)), a mycoses fungoides, a merkel cell cancer, a hematologic malignancy, a cancer of hematological tissues, a B cell cancer, a bronchus cancer, a stomach cancer, a brain or central nervous system cancer, a peripheral nervous system cancer, a uterine or endometrial cancer, a cancer of the oral cavity or pharynx, a liver cancer, a testicular cancer, a biliary tract cancer, a small bowel or appendix cancer, a salivary gland cancer, an adrenal gland cancer, an adenocarcinoma, an inflammatory myofibroblastic tumor, a gastrointestinal stromal tumor (GIST), a colon cancer, a myelodysplastic syndrome (MDS), a myeloproliferative disorder (MPD), a polycythemia Vera, a chordoma, a synovioma, an Ewing's tumor, a squamous cell carcinoma, a basal cell carcinoma, an adenocarcinoma, a sweat gland carcinoma, a sebaceous gland carcinoma, a papillary carcinoma, a papillary adenocarcinoma, a medullary carcinoma, a bronchogenic carcinoma, a renal cell carcinoma, a hepatoma, a bile duct carcinoma, a choriocarcinoma, a seminoma, an embryonal carcinoma, a Wilms' tumor, a bladder carcinoma, an epithelial carcinoma, a glioma, an astrocytoma, a medulloblastoma, a craniopharyngioma, an ependymoma, a pinealoma, a hemangioblastoma, an acoustic neuroma, an oligodendroglioma, a meningioma, a neuroblastoma, a retinoblastoma, a follicular lymphoma, a diffuse large B-cell lymphoma, a mantle cell lymphoma, a hepatocellular carcinoma, a thyroid cancer, a small cell cancer, an essential thrombocythemia, an agnogenic myeloid metaplasia, a hypereosinophilic syndrome, a systemic mastocytosis, a familiar hypereosinophilia, a neuroendocrine cancer, or a carcinoid tumor.

In a particular embodiment, the tumor is from a bladder or breast or colon or rectal or endometrial or kidney or leukemia or liver or lung or melanoma or non-Hodgkin lymphoma or pancreatic or prostate or thyroid cancer; more preferably the tumor is from an ovarian cancer or a breast cancer. In a particular embodiment, the tumor carries a BRCA1 mutation or a BRCA2 mutation; more preferably the BRCA1 or BRCA2 mutation from the tumor occurs in a LOH region or a non-LOH region.

In a particular embodiment, the tumor is a primary tumor or a metastasis.

The term "copy number alteration (CNA)", as used herein, refers to alteration in the number of copies of a nucleic acid sequence present in a test sample in comparison with the copy number of the nucleic acid sequence present in a reference sample.

A "copy number alteration" refers to the sequence of nucleic acid in which copy-number differences are found by comparison of a sequence of interest in test sample with an expected level of the sequence of interest. For example, the level of the sequence of interest in the test sample is compared to that present in a qualified sample. Copy number alterations include deletions, including microdeletions, insertions, including microinsertions, duplications, multiplications, inversions, translocations and complex multi-site variants. CNAs encompass chromosomal aneuploidies and partial aneuploidies.

A CNA is sometimes greater than or equal to a region of contiguous nucleotides in a reference genome of about 1 kilobase or greater in length (e.g., about 2, 3, 4, 5, 6, 7, 8, 9, 10, 20, 30, 40, 50, 60, 70, 80, 90, 100, 200, 300, 400, 500, 600, 700, 800, 900 kilobases, or greater, in length, or about 1, 2, 3, 4, 5, 6, 7, 8, 9, 10, 15, 20, 30, 40 or 50 megabases, or greater, in length). A CNA is about 7 megabases or greater in length (e.g., greater than or equal to about 8, 9, 10, 11, 12, 13, 14, 15, 16, 17, 18, 19, 20 megabases), and sometimes is less than about 7 megabases in length (e.g., less than about 1, 2, 3, 4, 5, 6 megabases).

In a particular embodiment, the CNA is a gain of a genomic region. In another particular embodiment, the CNA is a loss of a genomic region. In some embodiments, the CNA is a gain of a genomic region which results in that some genes of a given genomic region appear in the genome of the tumor in at least 3, 4, 5, 6 or more copies. In some embodiments, the CNA is a loss of a genomic region which results in that some genes of the genomic region appear as single copy in the genome of the cell or are absent.. In some embodiments, the first step comprises the determination of at least 2, at least 3, at least 4, at least 5, at least 6, at least 7, at least 8, at least 9, at least 10, at least 11 or the 12 genomic regions shown in Table 1.

The term "genomic region", as used herein, refers to a region in a human genome of which copy number, alone or in combination with other genomic regions, is correlated with a prediction of an effect.

The term "increased copy number" for a genomic region in the context of the method of the invention is understood as that at least one additional copy of the genomic region is present in the genome of tumor sample with respect to a reference sample. In some embodiments, the copy number of the genomic region under consideration is at least 3, at least 4, at least 5, at least 6, at least 7, at least 8, at least 9, at least 10 or more copies of the gene in the reference sample. The term "decreased copy number" in the context of the method of the invention is understood as that at least one less copy of the genomic region is present in the subject as compared to the copy number of the same genomic region in the reference sample.

In some embodiments, the copy number features comprise an absolute copy number feature. The absolute copy number may be computed for each genome segment and assigned a value. For example, the assigned values may include 0 (indicating a homozygous deletion), 1 (which may indicate a heterozygous deletion), 2 (which could be a normal count), or more (which may indicate copy number amplification). The absolute copy number feature may represent the actual copy number count (such as the average of the copy number for each segment analyzed) or a bin of copy number values. For example, copy numbers of at least 6 may be binned as representing a high copy number for a segment. Copy numbers between 3 and 5 may be binned as representing a moderately increased copy number. Copy numbers of 1 and 2 may be normal, and copy numbers of 0 may be binned as homozygous deletions.

Methods for determining CNA of a genomic region of interest are well-known in the art. Existing methods to determine CNA's typically include cytogenetic methods such as fluorescent in situ hybridization, comparative genomic hybridization, and/or virtual karyotyping with SNP arrays. Other methods include next-generation sequencing and quantitative PCR (qPCR), paralog-ratio testing (PRT) and molecular copy number counting (MCC). qPCR compares threshold cycles (Ct) between the target gene and a reference sequence with normal copy numbers, to generate ACt values which are used for CNA calculation. This method has been used in large-scale CNA analysis in detecting disease associations, for example, psoriasis and Crohn's disease. With the development of genome-wide CNA screening, qPCR is often used as a confirmation method for computationally identified loci. Other multiplex PCR-based approaches, such as multiplex amplifiable probe hybridization, multiplex ligation dependent probe amplification, multiplex PCR-based real-time invader assay, quantitative multiplex PCR of short fluorescent fragments, and multiplex amplicon quantification, have also been used for targeted screening and validation of CNAs.

In some embodiments, the tumor sample is a sample from a primary tumor or a metastasis. In yet another embodiment the tumor is an ovarian tumor, a breast tumor, a pancreatic tumor, a prostate tumor or a sarcoma.

In some embodiments, CNA variation in the genomic regions according to the invention is determined by whole genome sequencing. In some embodiments, CNA variation is determined by whole exome sequencing. Both the whole genome sequence and the whole exome sequence can be carried out by Next Generation Sequencing.

The method according to the present invention allows the prediction of whether the tumor has an HR status that can be considered as deficient. In some embodiments, the CNA in the at least one genomic region is determined in relation to a reference value (which may also be referred to herein as a threshold, threshold value, or the like). In some embodiments a CNA characterized by an increased copy number as compared to a reference or a CNA characterised by a reduced copy number as compared to a reference indicates HRD.

The reference or threshold value can determined using an appropriate measurement. In some embodiments, the reference value is determined for one or more control sample, such as a healthy control. In some embodiments, the threshold is determined using a statistical model, such as a machine learning model. As a non-limiting example, the threshold for DNA copy number determined using a statistical model, such as a machine learning model, can be more refined than simple comparison to diploid state, e g., by providing a more finely grained threshold value (perhaps due to technical resolution and/or biological causes) and/or a level of confidence. In some embodiments, the threshold is two copies, and any number of copies lower than two indicates a loss of copy number. Thus in some embodiments, the methods include detecting a number of copies below two, e.g., one (1) or zero (0) copy, in a sample from the tumor, and identifying the tumor who has 0 or 1 copy or a portion thereof, e.g., as described herein, as an HRD tumor. Tumours which are identified as having a gain of copy number (more than 2), can be identified as tumors having HRD. However, statistical analysis, including without limitation machine learning approaches, may determine an optimal reference value other than 2 to determine whether a tumor is deficient or not for homologous recombination.

In some embodiments, the method for determining the HR status of a tumor is applied to ovarian cancer and the at least one genomic region is selected from the group consisting of regions 2o, 3o, 6ο, 7o, 14o, 15o, 17o, 18o and 25o wherein:
- Region 2o comprises nucleotides 102100000 to 102900000 from chromosome 11
- Region 3o comprises nucleotides 121200000 to 127800000 from chromosome 11
- Region 6o comprises nucleotides 62300000 to 65700000 from chromosome 13
- Region 7o comprises nucleotides 79000000 to 87700000 from chromosome 13
- Region 14o comprises nucleotides 66800000 to 68700000 from chromosome 18,
- Region 15o comprises nucleotides 8700000 to 11800000 from chromosome 3,
- Region 17o comprises nucleotides 153000000 to 155000000 from chromosome 3,
- Region 18o comprises nucleotides 175700000 to179000000 from chromosome 3,
- Region 24o comprises nucleotides 13400000 to 15200000 from chromosome 6 and,
- Region 25o comprises nucleotides 1000000 to 2300000 from chromosome 6

In yet another embodiment, the method of the invention, when applied to ovarian cancer, comprises the determination of the genomic regions 6o and 25o, 3o and 14o, 2o and 3o, 2o and 7o, 15o and 24o, 14o and 24o, 2o and 24o, 15o and 25o, 2o and 15o, 3o and 7o, 6o and 14o, 3o and 15o, 2o and 6o, 3o and 24o, 2o and 25o, 3o and 25o, 3o and 6o, 14o and 25o or 2o and 14o.

In one embodiment, the method according to the present invention is carried out in a breast cancer and the at least one genomic region is selected from the group consisting of the 3b, 7b, 8b, 11b, 12b, 13b, 15b, 19b, 21b and 23b regions wherein:
- Region 3b comprises nucleotides 5400000 to 7000000 from chromosome 12,
- Region 7b comprises nucleotides 25000000 from 32700000 in chromosome 18,
- Region 8b comprises nucleotides 59000000 to 73100000 from chromosome 18,
- Region 11b comprises nucleotides 38700000 to 43400000 from chromosome 19,
- Region 12b comprises nucleotides 226100000 to 231000000 from chromosome 2,
- Region 13b comprises nucleotides 55000000 to 56500000 from chromosome 20,
- Region 15b comprises nucleotides 179000000 to 182700000 from chromosome 3,
- Region 19b comprises nucleotides 15200000 to 25200000 from chromosome 6,
- Region 21b comprising nucleotides 141000000 to 146364022 from chromosome 8 and
- Region 23b comprises nucleotides 14200000 to 16600000 from chromosome 9

In yet another embodiment, the method when applied to breast cancer, comprises s the determination of the genomic regions 8b and 15b, 8b and 12b, 12b and 23b, 7b and 15b, 7b and 12b, 15b and 23b, 3b and 8b, 8b and 11b, 11b and 23b, 8b and 23b, 3b and 12b, 12b and 15b, 3b and 15b, 3b and 7b, 8b and 19b, 7b and 23b, 3b and 11b, 7b and 11b or 3b and 23b.

Assaying the presence of CNA in a tumor may comprise the use of an algorithm or classifier. In some embodiments, assaying the presence or absence of CNA in a plurality of targets may comprise the use of a machine learning algorithm. The machine learning algorithm may comprise a supervised learning algorithm. Examples of supervised learning algorithms may include Average One-Dependence Estimators (AODE), Artificial neural network (e.g., Backpropagation), Bayesian statistics (e.g., Naive Bayes classifier, Bayesian network, Bayesian knowledge base), Case-based reasoning, Decision trees, Inductive logic programming, Gaussian process regression, Group method of data handling (GMDH), Learning Automata, Learning Vector Quantization, Minimum message length (decision trees, decision graphs, etc.), Lazy learning, Instance-based learning Nearest Neighbor Algorithm, Analogical modeling, Probably approximately correct learning (PAC) learning, Ripple down rules, a knowledge acquisition methodology, Symbolic machine learning algorithms, Subsymbolic machine learning algorithms, Support vector machines, Random Forests, Ensembles of classifiers, Bootstrap aggregating (bagging), and Boosting. Supervised learning may comprise ordinal classification such as regression analysis and Information fuzzy networks (IFN). In some embodiments, supervised learning methods may comprise statistical classification, such as AODE, Linear classifiers (e.g., Fisher's linear discriminant. Logistic regression, Naive Bayes classifier, Perceptron, and Support vector machine), quadratic classifiers, k-nearest neighbor, Boosting, Decision trees (e.g., C4.5, Random forests), Bayesian networks, and Hidden Markov models.

The machine learning algorithms may also comprise an unsupervised learning algorithm. Examples of unsupervised learning algorithms may include artificial neural network, Data clustering, Expectation-maximization algorithm. Self- organizing map. Radial basis function network. Vector Quantization, Generative topographic map, Information bottleneck method, and IBSEAD. Unsupervised learning may also comprise association rule learning algorithms such as Apriori algorithm, Eclat algorithm and FP-growth algorithm. Hierarchical clustering, such as Single-linkage clustering and Conceptual clustering, may also be used. In some embodiments, unsupervised learning may comprise partitional clustering such as K-means algorithm and Fuzzy clustering.

In some instances, the machine learning algorithms comprise a reinforcement learning algorithm. Examples of reinforcement learning algorithms include, but are not limited to, temporal difference learning, Q-learning and Learning Automata. In some embodiments, the machine learning algorithm may comprise Data Pre-processing.

Preferably, the machine learning algorithms may include, but are not limited to, Average One-Dependence Estimators (AODE), Fisher's linear discriminant, Logistic regression, Perceptron, Multilayer Perceptron, Artificial Neural Networks, Support vector machines, Quadratic classifiers, Boosting, Decision trees, C4.5, Bayesian networks, Hidden Markov models, High-Dimensional Discriminant Analysis, and Gaussian Mixture Models. The machine learning algorithm may comprise support vector machines, Naive Bayes classifier, k-nearest neighbor, high-dimensional discriminant analysis, or Gaussian mixture models. In some instances, the machine learning algorithm comprises Random Forests.

Once the presence of CNA in at least one genomic region according to the invention has been determined, the invention allows the determination of the HR status of the tumor, wherein said tumor can be deficient for HR or not.

In some embodiments, the determination of the CNA in at least one genomic region according to the invention allows the generation of a score (hereinafter CNA-score) which is a marker of the HR status of the tumor.

By comparing the HR status determined based on the CNA-score obtained according to the invention with the HR status determined by using any conventional method for the determination of the HR status. HR deficiency may be determined, for example, by the following: clinical phenotype; gene expression profiles of BRCAness or DNA repair, evaluating BRCA1 protein expression by immunohistochemistry, assessing tumor genome nucleotide sequences and mutational spectrums, or "sequence scars" characteristic of defective DNA repair via HR; targeted mutational profiling of HR genes using next- generation sequencing; BROCA targeted capture and massively parallel sequencing assay that identifies all types of mutations of key HR genes, including single-base substitutions, small insertions and deletions, and large gene rearrangements; determining the following scores: whole-genome tumor LOH scores, telomeric allelic imbalance (TAI) score, and large-scale state transitions (LST) score, and sensitivity to platinum compounds such as cisplatin; and/or, direct assessment of RAD51 foci formation via immunofluorescence or by assessing other DNA repair complexes via immunohistochemistry.

### Method for predicting tumor deficiency in the DNA homologous recombination (HR) pathway in a patient suffering from cancer

The inventive method according to the invention allows the use of the CNA or CNA combination to determine not only the HR status in a tumor sample but also to determine the HR status of a tumor in a cancer patient by analysing the genetic material of the patient.

Thus, in another aspect, the invention relates to an in vitro method for predicting tumor deficiency in the DNA homologous recombination (HR) pathway in a patient suffering from cancer, comprising determining in the genetic material of a sample from said patient the presence of CNA in at least one genomic region selected from the group consisting of the genomic regions as shown in Table 1, wherein the presence of said at least one CNA indicates that the tumor in the patient has a HR-deficient status.

The term "predicting tumor deficiency in the DNA homologous recombination (HR) pathway in a patient suffering from cancer" refers to the determination of whether the tumor in the cancer patient is HR deficient or not.

In some embodiment, the prediction results in that the tumor in the patient is characterized as HR deficient (HRD). A used herein the expressions "deficiency in the HR pathway" or "tumor deficiency in the HR pathway" are used interchangeably. Indeed, it refers to the genetic status of the tumor cells. However, in the case of germline mutations, said mutations can be found throughout the entire genome of the patient. As used herein the term "inactivation", when referring to a gene, can mean any type of deficiency of said gene. It encompasses germline mutations in the coding sequence, somatic mutations in the coding sequence, mutations in the promoter and methylation of the promoter.

The term "cancer", as used herein, is used interchangeably with "malignancy", "neoplasm", "tumor", and "carcinoma" and refers to cells that exhibit relatively abnormal, uncontrolled, and/or autonomous growth, so that they exhibit an aberrant growth phenotype characterized by a significant loss of control of cell proliferation. In some embodiments, a tumor may be or comprise cells that are precancerous (e.g., benign), malignant, pre-metastatic, metastatic, and/or non-metastatic. In some embodiments, a relevant cancer may be characterized by a solid tumor. In some embodiments, a relevant cancer may be characterized by a metastatic solid tumor. In some embodiments, a relevant cancer may be characterized by a hematologic tumor.

Suitable cancers to which the present invention can be applied are those defined in the context of the method for determining the HR status of the patient and are equally applicable to the present invention. In some embodiments, the cancer is ovarian, breast cancer, pancreatic cancer, prostate cancer or sarcoma.

The term ovarian cancer, as used herein, refers to a cancerous tumor of an ovary that may originate from the ovary itself or more commonly from communicating nearby structures such as fallopian tubes or the inner lining of the abdomen. Ovarian cancer include, without limitation, a germ cell tumor, a stromal tumor, a granulosa cell tumor. In some embodiments, a gynecologic cancer is associated with homologous recombination repair deficiency/homologous repair deficiency (HRD) and/or BRCA1/2 mutation(s). The term breast cancer, as used herein, refers to is cancer that develops from breast tissue and includes, without limitation, a hormone receptor (HR) positive breast cancer, including tumours that are estrogen receptor (ER) positive and/or progesterone positive (PR). The breast cancer may be HER positive breast cancer (HER+). The breast cancer may also include tumours that are ER+, PR+ and HER+. The breast cancer may also be a hormone receptor negative breast cancer, or a triple negative breast cancer, i.e., without ER, PR or overexpression of HER2.

The term pancreatic cancer, as used herein, refers to a group of malignancies affecting the pancreas and includes, without limitation, adenocarcinoma, neuroendocrine tumors or islet cell tumors, acinar cell carcinoma as well as other types of exocrine cancer such as adenosquamous carcinomas, squamous cell carcinomas, signet ring cell carcinomas, undifferentiated carcinomas, and undifferentiated carcinomas with giant cells. The term includes pancreatic cancers in Stages 0, IA, IB, IIA, IIB, III, and IV.

The term prostate cancer, as used herein, refer to a cancer that resides in prostate tissue. The prostate cancer can be benign, malignant or metastatic. The prostate cancer can be androgen-insensitive, hormone-resistant or castrate-resistant. The prostate cancer can be "advanced stage prostate cancer" or "advanced prostate cancer". Advanced stage prostate cancer includes a class of prostate cancers that has progressed beyond early stages of the disease. Types of advanced stage prostate cancers include, but are not limited to, metastatic prostate cancer, drug-resistant prostate cancer such as anti-androgen-resistant prostate cancer (e.g. enzalutamide-resistant prostate cancer, abiraterone- resistant prostate cancer, bicalutamide-resistant prostate cancer, etc.), taxane-resistant prostate cancer (e.g. docetaxel-resistant prostate cancer) and the like, hormone refractory prostate cancer, castration- resistant prostate cancer (CRPC), metastatic castration-resistant prostate cancer, AR-V7-induced drug- resistant prostate cancer such as AR-V7-induced anti-androgen-resistant prostate cancer (e.g. AR-V7-induced enzalutamide-resistant prostate cancer), AKRIC3-induced drug-resistant prostate cancer such as AKRIC3-induced anti-androgen-resistant prostate cancer (e.g. AKR1C3 -induced enzalutamide- resistant prostate cancer) and combinations thereof.

The term sarcoma, as used herein, generally refers to a tumor which is made up of a substance like the embryonic connective tissue and is generally composed of closely packed cells embedded in a fibrillar or homogeneous substance. The term, includes, without limitation, chondrosarcoma, fibrosarcoma, lymphosarcoma, melanosarcoma, myxosarcoma, osteosarcoma, Abemethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, or telangiectaltic sarcoma.

In one embodiment, the cancer is a cancer which has been previously characterized as HR deficient cancer or is suspected of being HR deficient.

As used herein ""HR Deficient cancer" refers to any cancer associated with or characterized by a HR deficiency. A HR deficiency may be associated with genetic or epigenetic alterations in the FA-BRCA pathway.

In some embodiments, the HR Deficient Cancer is associated with a HR deficiency associated with germline mutations of the tumor suppressors BRCA1 and BRCA2. In embodiments, the HR Deficient Cancer is associated with a HR deficiency associated with genetic and epigenetic inactivation of homologous recombination components broadly termed BRCAness.

In some embodiments, the HR Deficient Cancer is associated with BRCA1, BRCA2, dual BRCA1/BRCA2, PALB2, BARD1, BRIP1, RAD51C and/or RAD51D defects or mutations. In some embodiments, the HR Deficient Cancer is associated with PALB2, BARD1, BRIP1, RAD51C and/or RAD51D defects or mutations. In embodiments, the HR Deficient Cancer is associated with BRCA1, BRCA2, and/or dual BRCA1/BRCA2 defects or mutations. HR deficiency may be determined, for example, by the following: clinical phenotype; gene expression profiles of BRCAness or DNA repair, evaluating BRCA1 protein expression by immunohistochemistry, assessing tumor genome nucleotide sequences and mutational spectrums, or "sequence scars" characteristic of defective DNA repair via HR; targeted mutational profiling of HR genes using next- generation sequencing; BROCA targeted capture and massively parallel sequencing assay that identifies all types of mutations of key HR genes, including single-base substitutions, small insertions and deletions, and large gene rearrangements; determining the following scores: whole-genome tumor LOH scores, telomeric allelic imbalance (TAI) score, and large-scale state transitions (LST) score, and sensitivity to platinum compounds such as cisplatin; and/or, direct assessment of RAD51 foci formation via immunofluorescence or by assessing other DNA repair complexes via immunohistochemistry.

As used herein, the terms "patient", "subject" or "individual" can be an individual organism, a vertebrate, a mammal, or a human. In some embodiments, the subject, patient or individual is a human.

In some embodiments, CNA variation in the genomic regions according to the invention is determined by whole genome sequencing. In some embodiments, CNA variation is determined by whole exome sequencing. Both the whole genome sequence and the whole exome sequence can be carried out by Next Generation Sequencing.

The method according to the present invention allows the prediction of whether a tumor in a patient cancer has an HR status that can be considered as deficient. In some embodiments, the CNA in the at least one genomic region is determined in relation to a reference value (which may also be referred to herein as a threshold, threshold value, or the like). In some embodiments a CNA characterized by an increased copy number as compared to a reference or a CNA characterised by a reduced copy number as compared to a reference indicates HRD.

The reference or threshold value can determined using an appropriate measurement. In some embodiments, the reference value is determined for one or more control sample, such as a healthy control. In some embodiments, the threshold is determined using a statistical model, such as a machine learning model. As a non-limiting example, the threshold for DNA copy number determined using a statistical model, such as a machine learning model, can be more refined than simple comparison to diploid state, e g., by providing a more finely grained threshold value (perhaps due to technical resolution and/or biological causes) and/or a level of confidence. In some embodiments, the threshold is two copies, and any number of copies lower than two indicates a loss of copy number. Thus in some embodiments, the methods include detecting a number of copies below two, e.g., one (1) or zero (0) copy, in a sample from the tumor, and identifying the tumor who has 0 or 1 copy or a portion thereof, e.g., as described herein, as an HRD tumor. Tumours which are identified as having a gain of copy number (more than 2), can be identified as tumors having HRD. However, statistical analysis, including without limitation machine learning approaches, may determine an optimal reference value other than 2 to determine whether a tumor is deficient or not for homologous recombination.

In a particular embodiment the sample is a tumor sample. In yet another embodiment, the tumor sample is from a primary tumor or a metastasis. In another particular embodiment, the sample is a tumor sample which comprises: lymphoid islets in proximity to the tumor; lymph nodes located in proximity of the tumor; and/or adjacent normal tissue or blood from the periphery.

In another embodiment, the determination of the CNAs according to the inventive method is carried out in a sample that contains cell-free DNA.

"Cell-free DNA," "cfDNA molecules," or simply "cfDNA" include DNA molecules that naturally occur in a subject in extracellular form (e.g., in blood, serum, plasma, or other bodily fluids such as lymph, cerebrospinal fluid, urine, or sputum). While the cfDNA previously existed in a cell or cells in a large complex biological organism, e.g., a mammal, it has undergone release from the cell(s) into a fluid found in the organism, and may be obtained from a sample of the fluid without the need to perform an in vitro cell lysis step. cfDNA molecules may occur as DNA fragments.

In another particular embodiment the sample containing cell free DNA is any of the biofluids defined above. In yet another embodiment, the biofluid is blood or a derivative thereof. In yet another embodiment, the blood derivative is plasma or serum.

In another embodiment, the sample is a tumor sample with low tumor cellularity. The term "tumor cellularity", as used herein, refers to the relative content of tumor cells in the tumor sample with respect to the total number of cells. In a particular embodiment, the tumor cellularity in the sample is of about 40% or less, of about 35% or less, of about 30% or less, of about 25% or less, of about 20% or less, of about 15% or less, of about 10% or less or of about 5% or less.

It will be understood that in the method for determining the response of a cancer patient to an anti-cancer treatment, the biological sample that will be used for the detection of the CNA will be from the tumor which is causing the cancer for which the response is to be determined. Accordingly, if a patient is suffering from different cancer types, the response of each cancer type to the therapy will be determined using a sample from a tumor corresponding to the specific cancer for which the response to the therapy is to be predicted.

In another particular embodiment, the sample is obtained from the patient prior to the start of the antitumor treatment. In some embodiments, the sample is obtained from the patient at least 1 day, 2 days, 3 days, 4 days, 5 days, 6 days, 1 week, 2 weeks, 3 weeks, 1 months, 2 months, 3 months, 4 months, 5 months, 6 months, 7 months, 8 months, 9 months, 10 months, 11 months, 1 year or longer before the antitumor treatment is started.

In another particular embodiment, the sample is obtained from the patient after to the start of the antitumor treatment. In some embodiments, the anti-cancer treatment is started at least 1 day, 2 days, 3 days, 4 days, 5 days, 6 days, 1 week, 2 weeks, 3 weeks, 1 months, 2 months, 3 months, 4 months, 5 months, 6 months, 7 months, 8 months, 9 months, 10 months, 11 months, 1 year or longer after the sample has been obtained from the sample.

Once the sample is obtained, the presence of CNA in at least one genomic region is detected on the genetic material of the sample.

In some embodiments, the method for predicting tumor deficiency in the DNA homologous recombination (HR) pathway in a patient suffering from cancer is applied to ovarian cancer and the at least one genomic region is selected from the group consisting of regions 2o, 3o, 6o, 7o, 14o, 15o, 17o, 18o and 25o wherein:
- Region 2o comprises nucleotides 102100000 to 102900000 from chromosome 11
- Region 3o comprises nucleotides 121200000 to 127800000 from chromosome 11
- Region 6o comprises nucleotides 62300000 to 65700000 from chromosome 13
- Region 7o comprises nucleotides 79000000 to 87700000 from chromosome 13
- Region 14o comprises nucleotides 66800000 to 68700000 from chromosome 18,
- Region 15o comprises nucleotides 8700000 to 11800000 from chromosome 3,
- Region 17o comprises nucleotides 153000000 to 155000000 from chromosome 3,
- Region 18o comprises nucleotides 175700000 to179000000 from chromosome 3,
- Region 24o comprises nucleotides 13400000 to 15200000 from chromosome 6 and,
- Region 25o comprises nucleotides 1000000 to 2300000 from chromosome 6

In yet another embodiment, the method of the invention, when applied to ovarian cancer, comprises the determination of the genomic regions 6o and 25o, 3o and 14o, 2o and 3o, 2o and 7o, 15o and 24o, 14o and 24o, 2o and 24o, 15o and 25o, 2o and 15o, 3o and 7o, 6o and 14o, 3o and 15o, 2o and 6o, 3o and 24o, 2o and 25o, 3o and 25o, 3o and 6o, 14o and 25o or 2o and 14o.

In some embodiments, the method for predicting tumor deficiency in the DNA homologous recombination (HR) pathway in a patient suffering from cancer is applied to breast cancer and the at least one genomic region is selected from the group consisting of the 3b, 7b, 8b, 11b, 12b, 13b, 15b, 19b, 21b and 23b regions wherein:
- Region 3b comprises nucleotides 5400000 to 7000000 from chromosome 12,
- Region 7b comprises nucleotides 25000000 from 32700000 in chromosome 18,
- Region 8b comprises nucleotides 59000000 to 73100000 from chromosome 18,
- Region 11b comprises nucleotides 38700000 to 43400000 from chromosome 19,
- Region 12b comprises nucleotides 226100000 to 231000000 from chromosome 2,
- Region 13b comprises nucleotides 55000000 to 56500000 from chromosome 20,
- Region 15b comprises nucleotides 179000000 to 182700000 from chromosome 3,
- Region 19b comprises nucleotides 15200000 to 25200000 from chromosome 6,
- Region 21b comprising nucleotides 141000000 to 146364022 from chromosome 8 and
- Region 23b comprises nucleotides 14200000 to 16600000 from chromosome 9

In yet another embodiment, the method when applied to breast cancer, comprises s the determination of the genomic regions 8b and 15b, 8b and 12b, 12b and 23b, 7b and 15b, 7b and 12b, 15b and 23b, 3b and 8b, 8b and 11b, 11b and 23b, 8b and 23b, 3b and 12b, 12b and 15b, 3b and 15b, 3b and 7b, 8b and 19b, 7b and 23b, 3b and 11b, 7b and 11b or 3b and 23b.

### Method for predicting response of a cancer patient to chemotherapy

The inventive methods according to the invention allows the use of the CNA or CNA combination not only as surrogate markers for the HRD status of the tumour, but also for predicting the response of the tumour to treatments that are known to be particularly effective in tumors having homologous recombination defects, such as platinum-based chemotherapy and PARPi. Moreover, the method for determining HRD status of tumor developed by the inventors is particularly advantageous in that it can determine the HRD status in a liquid biopsy, allowing a less invasive determination.

Accordingly, in a third aspect, the present invention relates to an in vitro method for predicting the response of a cancer patient to an antitumor treatment, said method comprising determining in the genetic material of a sample from said patient the presence of CNA in at least one genomic region selected from the group consisting of the genomic regions as shown in Table 1, wherein the presence of said at least one CNA indicates that the cancer is responsive to an antitumor treatment selected from the group consisting of a chemotherapy, targeted therapy or immunotherapy.

The term "predicting the response or a cancer patient", as used herein, is intended to mean assessing the likelihood that a patient will experience a positive or negative outcome with a particular treatment. As used herein, indicative of a positive treatment outcome refers to an increased likelihood that the patient will experience beneficial results from the selected treatment (e.g., complete or partial remission, reduced tumour size, etc.). Indicative of a negative treatment outcome is intended to mean an increased likelihood that the patient will not benefit from the selected treatment with respect to the progression of the underlying cancer.

In some embodiment, the response of the patient is predicted as good prognosis.

"Good prognosis" refers to a normal risk of relapse, a reduced risk of relapse, an increased chance for remission, an increased relapse-free survival time, or a high survival rate. In embodiments, a "good prognosis" refers to a reduced risk of relapse, an increased chance for remission, an increased relapse-free survival time, or a high survival rate. In embodiments, a "good prognosis" refers to a reduced risk of relapse. In embodiments, a "good prognosis" refers to an increased chance for remission. In embodiments, a "good prognosis" refers to an increased relapse-free survival time. In embodiments, a "good prognosis" refers to a high survival rate. In embodiments, a high survival rate refers to a 5- year survival rate greater than 50 percent. In embodiments, a high survival rate refers to a 5-year survival rate greater than 60 percent, greater than 70 percent, greater than 80 percent, or greater than 90 percent. In embodiments, "good prognosis" is an increased likelihood of a good prognosis.

The terms "cancer" has been defined in the context of the second method of the invention and applies equally to the method for predicting the response of a patient to an anti-cancer treatment. In some embodiments, a relevant cancer may be characterized by a metastatic solid tumor. In some embodiments, a relevant cancer may be characterized by a hematologic tumor.

Suitable cancers to which the present invention can be applied are those defined in the context of the method for determining the HR status of the patient and in the context predicting tumor deficiency in the DNA homologous recombination (HR) pathway in a patient suffering from cancer. In some embodiments, the cancer is ovarian, breast cancer, pancreatic cancer, prostate cancer or sarcoma.

The terms ovarian cancer, breast cancer, pancreatic cancer, prostate cancer, sarcoma have been defined in the context of the second method of the invention and applies equally to the method for predicting the response of a patient to an anti-cancer treatment.

In one embodiment, the cancer is a cancer which has been previously characterized as HR deficient cancer or is suspected of being HR deficient. The term "HR Deficient cancer" has been defined in the context of the second method of the invention and applies equally to the present method

As used herein, the terms "patient", "subject" or "individual" can be an individual organism, a vertebrate, a mammal, or a human. In some embodiments, the subject, patient or individual is a human.

The method according to the present invention allows the prediction of a response of a cancer in a patient to an anti-tumor treatment.

The term "antitumor treatment", as used herein, refers to a treatment against cancer. The types of treatment that the patient will receive will depend on the type of cancer and how advanced it its. In a particular embodiment, the antitumor treatment is selected from a group consisting of chemotherapy, hormone therapy, hyperthermia, immunotherapy, photodynamic therapy, radiation therapy, stem cell transplant, surgery or targeted therapy; more preferably, the antitumor treatment is selected from the group consisting of a chemotherapy, targeted therapy or immunotherapy.

The term "radiation therapy" or "radiotherapy" refers to a therapy that uses ionizing radiation, and is generally provided as part of cancer treatment to control or kill malignant cells. Radiation therapy is normally delivered by a linear accelerator. Radiation therapy may be curative in a number of types of cancer if they are localized to one area of the body. It may also be used as part of adjuvant therapy, to prevent tumor recurrence after surgery that removes a primary malignant tumor. Radiation therapy is synergistic with chemotherapy, and has been used before, during, and after chemotherapy in susceptible cancers.

"Hormone therapy", as used herein, is a treatment that adds, blocks, or removes hormones. Hormones can also cause certain cancers (such as prostate and breast cancer) to grow. In the context of the present invention "hormone therapy" describes a treatment aiming to slow or stop the growth of cancer, synthetic hormones or other drugs may be given to block the body's natural hormones, or surgery is used to remove the gland that makes a certain hormone

"Photodynamic therapy" as used herein refers to therapy which uses certain chemicals known as photosensitizing compounds to treat or prevent cancers. Examples of photodynamic therapy include treatment with compounds, such as Visudyne^{™} and porfimer sodium.

The term "hyperthermia", as used herein, refers to the treatment by locally applying d temperatures higher than the body temperature, in particular above 37 degrees centigrade in humans, including a local body temperature comprised between 37.5 degrees centigrade and 45 degrees centigrade, preferably 39 and 45 degrees centigrade Hyperthermia will allow eliminating or damaging the target cells or sensitising them for another treatment, especially radiotherapy or chemotherapy.

The term "chemotherapy" refers to a type of cancer treatment that uses one or more anti-cancer drugs (chemotherapeutic agents) as part of a standardized chemotherapy regimen. Chemotherapy may be given with a curative intent (which almost always involves combinations of drugs), or it may aim to prolong life or to reduce symptoms (palliative chemotherapy). Chemotherapy drugs include, but are not limited to, alkylating agents, nitrosoureas, antimetabolites, alkaloids, antitumor antibiotics, hormonal agents and biological response modifiers. Examples of chemotherapy drugs include, but are not limited to, cyclophosphamide melphalan. temozolomide. carboplatin, cisplatin, oxaliplatin, 5- fluorouracil, 6-mercaptopurine, cytarabine, gemcitabine, methotrexate, actimycin-D, blemycin, daunorubicin, doxorubicin, docetaxel, estramustine, paclitaxel, vinblastine, etoposide, irinotecan, teniposide, topotecan, prednisone, methylprednisolone and dexamethasone.

The term "targeted therapy", as used herein, a "targeted therapy" refers to a method for treating cancer that blocks the growth of cancer cells by interfering with specific targeted molecules needed for carcinogenesis and tumor growth, rather than by simply interfering with all rapidly dividing cells. Exemplary forms of targeted therapy include, but are not limited to, antibody-drug conjugates, nano-engineered enzymes that bind to a tumor cell, and chemical entities that target or preferentially target a protein or enzyme that carries a mutation or other genetic alteration that is specific to cancer cells and is not found in normal host tissue. Exemplary drugs that are used in targeted therapy include, but are not limited to, trastuzumab and bevacizumab

The term "immunotherapy" refers to methods of treating cancer that are based on the stimulation of the patient's immune system. Cancer immunotherapy exploits the fact that cancer cells often have tumor antigens that can be detected and bound by the antibodies of the immune system. Clinical success of cancer immunotherapy is highly variable between different forms of cancer. Examples of immunotherapy include, but are not limited to, therapeutic cancer vaccines, CAR-T cell, and targeted antibody therapies. Examples of drugs used in immunotherapy include, but are not limited to, ipilimumab, pembrolizumab, nivolumab and atezolizumab. In embodiments, the immunotherapy includes administration of an effective amount of an anticancer agent as set forth herein.

The determination of the CNA or CNA combination allows predicting the response of the tumour to treatments that are known to be particularly effective in tumors having homologous recombination defects, preferably a therapy comprising a PARP inhibitor and/or platinum-based chemotherapy.

In one embodiment, the method of the invention allows the determination of the response of a patient to a treatment by chemotherapy using a platinum-based drugs.

The term "platinum-based drug", as used herein, refers to coordination complexes of platinum. Examples of these type of drugs include cisplatin, oxaliplatin, carboplatin, nedaplatin, triplatin tetranitrate, phenanthriplatin, picoplatin and satraplatin.

In one embodiment, the method of the invention allows the determination of the response of a patient to a treatment by chemotherapy using a PARP inhibitor.

The term "PARPi", as used herein, refers to PARP inhibitors, a group of pharmacological inhibitors of the enzyme poly ADP ribose polymerase (PARP). They are used in the treatment of cancer. PARP inhibitors appear to improve progression-free survival in woman with recurrent platinum sensitive ovarian cancer. Examples of these type of drugs include olaparib, rucaparib, niraparib and talazoparib.

The term "sample" or "biological sample", as used herein, refers to biological material isolated from a subject. The biological sample contains any biological material suitable for detecting cancer. In a particular embodiment, the sample is either a tumor sample or a biofluid. The sample can be isolated from any suitable tissue or biological fluid such as, for example blood, saliva, plasma, serum, urine, cerebrospinal liquid (CSF), feces, a surgical specimen, a specimen obtained from a biopsy, and a tissue sample embedded in paraffin. Methods for isolating samples are well known to those skilled in the art.

In a particular embodiment the sample is a tumor sample. In yet another embodiment, the tumor sample is from a primary tumor or a metastasis. In another particular embodiment, the sample is a tumor sample which comprises: lymphoid islets in proximity to the tumor; lymph nodes located in proximity of the tumor; and/or adjacent normal tissue or blood from the periphery.

In another embodiment, the determination of the CNAs according to the inventive method is a carried out in a sample that contains cell-free DNA.

"Cell-free DNA," "cfDNA molecules," or simply "cfDNA" include DNA molecules that naturally occur in a subject in extracellular form (e.g., in blood, serum, plasma, or other bodily fluids such as lymph, cerebrospinal fluid, urine, or sputum). While the cfDNA previously existed in a cell or cells in a large complex biological organism, e.g., a mammal, it has undergone release from the cell(s) into a fluid found in the organism, and may be obtained from a sample of the fluid without the need to perform an in vitro cell lysis step. cfDNA molecules may occur as DNA fragments.

In another particular embodiment the sample containing cell free DNA is any of the biofluids defined above. In yet another embodiment, the biofluid is blood or a derivative thereof. In yet another embodiment, the blood derivative is plasma or serum.

In another embodiment, the sample is a tumor sample with low tumor cellularity. The term "tumor cellularity", as used herein, refers to the relative content of tumor cells in the tumor sample with respect to the total number of cells. In a particular embodiment, the tumor cellularity in the sample is of about 40% or less, of about 35% or less, of about 30% or less, of about 25% or less, of about 20% or less, of about 15% or less, oft about 10% or less or of about 5% or less.

It will be understood that in the method for determining the response of a cancer patient to an anti-cancer treatment, the biological sample that will be used for the detection of the CNA will be from the tumor which is causing the cancer for which the response is to be determined. Accordingly, if a patient is suffering from different cancer types, the response of each cancer type to the therapy will be determined using a sample from a tumor corresponding to the specific cancer for which the response to the therapy is to be predicted.

In another particular embodiment, the sample is obtained from the patient prior to the start of the antitumor treatment. In some embodiments, the sample is obtained from the patient at least 1 day, 2 days, 3 days, 4 days, 5 days, 6 days, 1 week, 2 weeks, 3 weeks, 1 months, 2 months, 3 months, 4 months, 5 months, 6 months, 7 months, 8 months, 9 months, 10 months, 11 months, 1 year or longer before the antitumor treatment is started.

In another particular embodiment, the sample is obtained from the patient after to the start of the antitumor treatment. In some embodiments, the anti-cancer treatment is started at least 1 day, 2 days, 3 days, 4 days, 5 days, 6 days, 1 week, 2 weeks, 3 weeks, 1 months, 2 months, 3 months, 4 months, 5 months, 6 months, 7 months, 8 months, 9 months, 10 months, 11 months, 1 year or longer after the sample has been obtained form the sample.

Once the sample is obtained, the presence of CNA in at least one genomic region is detected on the genetic material of the sample.

In some embodiments, the method for predicting the response in a patient is carried out in a patient suffering from ovarian cancer, in which case the at least one genomic region is selected from the group consisting of regions 2o, 3o, 6o, 7o, 14o, 15o, 17o, 18o and 25o wherein:
- Region 2o comprises nucleotides 102100000 to 102900000 from chromosome 11
- Region 3o comprises nucleotides 121200000 to 127800000 from chromosome 11
- Region 6o comprises nucleotides 62300000 to 65700000 from chromosome 13
- Region 7o comprises nucleotides 79000000 to 87700000 from chromosome 13
- Region 14o comprises nucleotides 66800000 to 68700000 from chromosome 18,
- Region 15o comprises nucleotides 8700000 to 11800000 from chromosome 3,
- Region 17o comprises nucleotides 153000000 to 155000000 from chromosome 3,
- Region 18o comprises nucleotides 175700000 to179000000 from chromosome 3,
- Region 24o comprises nucleotides 13400000 to 15200000 from chromosome 6 and,
- Region 25o comprises nucleotides 1000000 to 2300000 from chromosome 6

In yet another embodiment, the method for predicting the response in a patient according to the invention, when applied to ovarian cancer, comprises the determination of the genomic regions 6o and 25o, 3o and 14o, 2o and 3o, 2o and 7o, 15o and 24o, 14o and 24o, 2o and 24o, 15o and 25o, 2o and 15o, 3o and 7o, 6o and 14o, 3o and 15o, 2o and 6ο, 3o and 24o, 2o and 25o, 3o and 25o, 3o and 6ο, 14o and 25o or 2o and 14o.

In one embodiment, the method for predicting the response in a patient according to the present invention is carried out in a patient suffering from breast cancer and the at least one genomic region is selected from the group consisting of the 3b, 7b, 8b, 11b, 12b, 13b, 15b, 19b, 21b and 23b regions wherein:
- Region 3b comprises nucleotides 5400000 to 7000000 from chromosome 12,
- Region 7b comprises nucleotides 25000000 from 32700000 in chromosome 18,
- Region 8b comprises nucleotides 59000000 to 73100000 from chromosome 18,
- Region 11b comprises nucleotides 38700000 to 43400000 from chromosome 19,
- Region 12b comprises nucleotides 226100000 to 231000000 from chromosome 2,
- Region 13b comprises nucleotides 55000000 to 56500000 from chromosome 20,
- Region 15b comprises nucleotides 179000000 to 182700000 from chromosome 3,
- Region 19b comprises nucleotides 15200000 to 25200000 from chromosome 6,
- Region 21b comprising nucleotides 141000000 to 146364022 from chromosome 8 and
- Region 23b comprises nucleotides 14200000 to 16600000 from chromosome 9

In yet another embodiment, the method for predicting the response of a patient, when applied to a breast cancer patient, comprises the determination of the genomic regions 8b and 15b, 8b and 12b, 12b and 23b, 7b and 15b, 7b and 12b, 15b and 23b, 3b and 8b, 8b and 11b, 11b and 23b, 8b and 23b, 3b and 12b, 12b and 15b, 3b and 15b, 3b and 7b, 8b and 19b, 7b and 23b, 3b and 11b, 7b and 11b or 3b and 23b.

It will be understood that the determination of the presence of CNAs in the sample can be carried out using any method known in the art, such as whole genome sequencing (WGS), whole exome sequencing (WES), whole transcriptome sequencing (WTS) targeted region sequencing, ChIP sequencing (CHip-seq), ATAC sequencing (ATAC-seq). In a preferred embodiment, the determination of the presence of the CNAs is carried out by shallow whole genome sequencing (shWGS).

In some embodiments, the CNA in at least one genomic region is determined in relation to a reference value (which may also be referred to herein as a threshold, threshold value, or the like). In some embodiments a CNA characterized by an increased copy number as compared to a reference indicates potential benefit of a PARP inhibitor and/or of benefit of platinum-based chemotherapy. Similarly, a CNA characterised by a reduced copy number as compared to a reference can indicate potential benefit of a PARP inhibitor and/or of benefit of platinum-based chemotherapy.

The reference or threshold value can determined using an appropriate measurement. In some embodiments, the reference value is determined for one or more control sample, such as a healthy control. In some embodiments, the threshold is determined using a statistical model, such as a machine learning model. As a non-limiting example, the threshold for DNA copy number determined using a statistical model, such as a machine learning model, can be more refined than simple comparison to diploid state, e g., by providing a more finely grained threshold value (perhaps due to technical resolution and/or biological causes) and/or a level of confidence. In some embodiments, the threshold is two copies, and any number of copies lower than two indicates a loss of copy number. Thus in some embodiments, the methods include detecting a number of copies below two, e.g., one (1) or zero (0) copy, in a sample from a subject, and identifying a subject who has 0 or 1 copy or a portion thereof, e.g., as described herein, as a subject who is likely to respond to PARP inhibitors or platinum-based chemotherapy. Subjects who are identified as having a gain of copy number (more than 2), can be identified as a subject who is likely to respond to PARP inhibitor therapy or platinum-based chemotherapy. Subjects who are identified as a likely to respond to PARP inhibitor therapy or platinum-based chemotherapy can then be be administered a treatment comprising PARP inhibitors and/or a platinum-based chemotherapy (and optionally another therapy (e g., radiotherapy, immunotherapy, alternate chemotherapy, or surgical resection). However, statistical analysis, including without limitation machine learning approaches, may determine an optimal reference value other than 2 to determine responders or non-responders.

### Methods for personalized therapy

In another aspect, the invention relates to a method for the treatment of a cancer patient which comprises:
(i) predicting the response of a patient to an antitumor treatment according to the invention and
(ii) treating the patient with an antitumor treatment selected from the group consisting of a chemotherapy, targeted therapy or immunotherapy is the patient is identified in step (i) as being responsive to the tumor treatment.
   (i) Predicting the tumor deficiency in the DNA homologous recombination (HR) pathway in the patient suffering from cancer or predicting the response of the cancer to an antitumor treatment by the method according to the invention and
   (ii) treating the patient with an antitumor treatment selected from the group consisting of a chemotherapy, targeted therapy or immunotherapy if the patient is determined in step (i) as having a tumor deficiency in the DNA homologous recombination (HR) pathway or predicted in step (i) as being responsive to the tumor treatment.

The term "treatment", as used herein comprises any type of therapy, which aims at terminating, preventing, ameliorating and/or reducing the susceptibility to a clinical condition as described herein. In a preferred embodiment, the term treatment relates to prophylactic treatment (i.e. a therapy to reduce the susceptibility of a clinical condition, a disorder or condition as defined herein). Thus, "treatment," "treating," and the like, as used herein, refer to obtaining a desired pharmacologic and/or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, and/or immune deficiency.

The method for personalized therapy according to the invention comprises, in a first step, predicting the tumor deficiency in the DNA homologous recombination (HR) pathway in the patient suffering from cancer or predicting the response of the patient to an anticancer therapy using the methods according to the invention. All the embodiments of the methods for predicting a tumor deficiency in the DNA homologous recombination (HR) pathway in the patient suffering from cancer or for predicting the response of the patient to an anticancer therapy have been explained in detail above and are equally applicable to the method for personalized therapy according to the invention.

In some embodiments, the sample is any sample that contains genetic material from the tumor, which includes tumor samples and biofluid samples. In some embodiments, wherein the sample is a tumor sample, then the tumor sample is a sample from a primary tumor or a metastasis. In some embodiments, wherein the sample is a biofluid, the biofluid is blood or a derivative thereof.

In some embodiment, the cancer patient suffers from, but not limited to, ovarian, breast cancer, pancreatic and prostate cancer.

In some embodiments, the sample is obtained from the patient prior to the start of the antitumor treatment or wherein the sample is obtained from the patient after the start of the antitumor treatment.

In some embodiments, the prediction of the response in a patient is carried out in a patient suffering from ovarian cancer, in which case the at least one genomic region is selected from the group consisting of regions 2o, 3o, 6ο, 7o, 14o, 15o, 17o, 18o and 25o wherein:
- Region 2 comprises nucleotides 102100000 to 102900000 from chromosome 11
- Region 3 comprises nucleotides 121200000 to 127800000 from chromosome 11
- Region 6 comprises nucleotides 62300000 to 65700000 from chromosome 13
- Region 7 comprises nucleotides 79000000 to 87700000 from chromosome 13
- Region 14 comprises nucleotides 66800000 to 68700000 from chromosome 18,
- Region 15 comprises nucleotides 8700000 to 11800000 from chromosome 3,
- Region 17 comprises nucleotides 153000000 to 155000000 from chromosome 3,
- Region 18 comprises nucleotides 175700000 to179000000 from chromosome 3,
- Region 24 comprises nucleotides 13400000 to 15200000 from chromosome 6 and,
- Region 25 comprises nucleotides 1000000 to 2300000 from chromosome 6

In yet another embodiment, the prediction of the response in a patient, when applied to ovarian cancer, comprises the determination of the genomic regions 6o and 25o, 3o and 14o, 2o and 3o, 2o and 7o, 15o and 24o, 14o and 24o, 2o and 24o, 15o and 25o, 2o and 15o, 3o and 7o, 6o and 14o, 3o and 15o, 2o and 6o, 3o and 24o, 2o and 25o, 3o and 25o, 3o and 6o, 14o and 25o or 2o and 14o.

In one embodiment, the prediction of the response in a patient according to the present invention is carried out in a patient suffering from breast cancer and the at least one genomic region is selected from the group consisting of the 3b, 7b, 8b, 11b, 12b, 13b, 15b, 19b, 21b and 23b regions wherein:
- Region 3b comprises nucleotides 5400000 to 7000000 from chromosome 12,
- Region 7b comprises nucleotides 25000000 from 32700000 in chromosome 18,
- Region 8b comprises nucleotides 59000000 to 73100000 from chromosome 18,
- Region 11b comprises nucleotides 38700000 to 43400000 from chromosome 19,
- Region 12b comprises nucleotides 226100000 to 231000000 from chromosome 2,
- Region 13b comprises nucleotides 55000000 to 56500000 from chromosome 20,
- Region 15b comprises nucleotides 179000000 to 182700000 from chromosome 3,
- Region 19b comprises nucleotides 15200000 to 25200000 from chromosome 6,
- Region 21b comprising nucleotides 141000000 to 146364022 from chromosome 8 and
- Region 23b comprises nucleotides 14200000 to 16600000 from chromosome 9

In yet another embodiment, the prediction in the response of a patient, when applied to a breast cancer patient, comprises the determination of the genomic regions 8b and 15b, 8b and 12b, 12b and 23b, 7b and 15b, 7b and 12b, 15b and 23b, 3b and 8b, 8b and 11b, 11b and 23b, 8b and 23b, 3b and 12b, 12b and 15b, 3b and 15b, 3b and 7b, 8b and 19b, 7b and 23b, 3b and 11b, 7b and 11b or 3b and 23b.

In another embodiment, the CNA is a gain of a genomic region or a loss of a genomic region. In yet another embodiment, the determination of the presence of said at least one CNA is carried out by whole genome sequencing. In yet another embodiment, the whole genome sequencing is carried out by shallow whole genome sequencing (shWGS).

In a second step, the method for personalized therapy according to the invention comprises treating the patient which has been identified as being responsive to the tumor treatment with an antitumor treatment selected from the group consisting of a chemotherapy, hormone therapy, hyperthermia, immunotherapy, photodynamic therapy, radiation therapy, stem cell transplant, surgery or targeted therapy; more preferably, the antitumor treatment is selected from the group consisting of a chemotherapy, targeted therapy or immunotherapy.

The terms and expressions "chemotherapy", "hormone therapy", "hyperthermia", "immunotherapy", "photodynamic therapy", "radiation therapy", "stem cell transplant", "surgery or "targeted therapy" have been defined in relation to the method for the prediction of the response of a patient to an anticancer therapy and are equally applicable to the method for treatment according to the present invention.

In a preferred embodiment, the antitumor treatment is a treatment that comprises a platinum-based drug or a treatment that comprises a PARPi.

The term "platinum-based drug" and "PARPi" have been defined in the context of the method for predicting the response of a cancer patient according to the invention and are equally applicable to the personalized medicine method as defined herein.

### Computer systems and programs configured for carrying out the methods of the invention

The methods described herein may be implemented using one or more computer systems. Accordingly, in another aspect, the invention relates to a computer system, comprising: one or more processors; a memory; and one or more programs, wherein the one or more programs are stored in the memory and configured to be executed by the one or more processors, the one or more programs including instructions for: performing the method of the invention

Such computer systems can include one or more programs configured to execute one or more processors for the computer system to perform such methods. One or more steps of the computer-implemented methods may be performed automatically. The computer system may include one or more computing nodes. For example, a system may include two or more computing nodes (e.g., servers, computers, routers, or other types of electronic devices that include a network interface), which may be connected and configured to communicate and execute the methods over said network on one or more computing nodes of the network.

In another aspect, the invention relates to a non-transitory computer-readable storage medium storing one or more programs, the one or more programs comprising instructions, which when executed by one or more processors of an electronic device, cause the electronic device to perform the method of the invention.

Storage can be any suitable device that provides storage, such as an electrical, magnetic or optical memory including RAM, cache, hard drive, or removable storage disk.

The one or more programs comprising instructions for performing the method of the invention can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport readable medium can include, but is not limited to, an electronic, magnetic, optical, electromagnetic or infrared wired or wireless propagation medium.

The storage medium may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

### Further aspects of the invention

1. An *in vitro* method for determining the DNA homologous recombination (HR) status of a tumor, comprising determination in a biological sample of said tumor the presence of a copy number alteration (CNA) in at least one genomic region, wherein the genomic region is selected from the group consisting of the genomic regions as shown in Table 1 wherein the presence of said at least one CNA in at least one genomic region is correlated with a HR-deficient status.
2. The method according to aspect 1 wherein the tumor sample is a sample from a primary tumor or a metastasis.
3. An in vitro method for predicting tumor deficiency in the DNA homologous recombination (HR) pathway in a patient suffering from cancer, comprising determining in the genetic material of a sample from said patient the presence of CNA in at least one genomic region selected from the group consisting of the genomic regions as shown in Table 1, wherein the presence of said at least one CNA indicates that the tumor in the patient has a HR-deficient status.
4. The method according to aspect 3 wherein the sample is either a tumor sample or a biofluid sample.
5. The method according to aspect 4 wherein the tumor sample is a sample from a primary tumor or a metastasis.
6. The method according to aspect 4 wherein the biofluid is blood or a derivative thereof.
7. The method according to any of aspects 3 to 6 wherein the cancer patient suffers from, but not limited to, ovarian, breast cancer, pancreatic cancer, prostate cancer or sarcoma.
8. The method according to aspect 7 wherein the cancer patient suffers from ovarian or breast cancer
9. The method according to any of aspects 3 to 8 wherein the cancer is an HR-deficient cancer or is cancer that is suspected of being HR deficient.
10. An *in vitro* method for predicting the response of a cancer patient to an antitumor treatment, said method comprising determining in the genetic material of a sample from said patient the presence of CNA in at least one genomic region selected from the group consisting of the genomic regions as shown in Table 1, wherein the presence of said at least one CNA indicates that the cancer is responsive to an antitumor treatment selected from the group consisting of a chemotherapy, targeted therapy or immunotherapy.
11. The method of aspect 10 wherein the antitumor treatment is a treatment that comprises platinum-based drugs or a treatment that comprises a PARPi.
12. The method according to aspect 11 wherein the sample is obtained from the patient prior to the start of the antitumor treatment or wherein the sample is obtained from the patient after the start of the antitumor treatment.
13. The method according to any preceding aspect wherein the method comprises the determination of the CNA in at least two genomic regions.
14. The method according to any preceding aspect wherein the cancer is ovarian cancer and wherein the at least one genomic region is selected from the group consisting of regions 2o, 3o, 6o, 7o, 14o, 15o, 17o, 18o and 25o wherein:
   - Region 2 comprises nucleotides 102100000 to 102900000 from chromosome 11
   - Region 3 comprises nucleotides 121200000 to 127800000 from chromosome 11
   - Region 6 comprises nucleotides 62300000 to 65700000 from chromosome 13
   - Region 7 comprises nucleotides 79000000 to 87700000 from chromosome 13
   - Region 14 comprises nucleotides 66800000 to 68700000 from chromosome 18,
   - Region 15 comprises nucleotides 8700000 to 11800000 from chromosome 3,
   - Region 17 comprises nucleotides 153000000 to 155000000 from chromosome 3,
   - Region 18 comprises nucleotides 175700000 to179000000 from chromosome 3,
   - Region 24 comprises nucleotides 13400000 to 15200000 from chromosome 6 and,
   - Region 25 comprises nucleotides 1000000 to 2300000 from chromosome 6
15. The method according to aspect 14 wherein the method comprises the determination of the genomic regions 6o and 25o, 3o and 14o, 2o and 3o, 2o and 7o, 15o and 24o, 14o and 24o, 2o and 24o, 15o and 25o, 2o and 15o, 3o and 7o, 6o and 14o, 3o and 15o, 2o and 6o, 3o and 24o, 2o and 25o, 3o and 25o, 3o and 6o, 14o and 25o or 2o and 14o.
16. The method according to any preceding aspects wherein the cancer is breast cancer and wherein the at least one genomic region is selected from the group consisting of the 3b, 7b, 8b, 11b, 12b, 13b, 15b, 19b, 21b and 23b regions wherein:
   - Region 3b comprises nucleotides 5400000 to 7000000 from chromosome 12,
   - Region 7b comprises nucleotides 25000000 from 32700000 in chromosome 18,
   - Region 8b comprises nucleotides 59000000 to 73100000 from chromosome 18,
   - Region 11b comprises nucleotides 38700000 to 43400000 from chromosome 19,
   - Region 12b comprises nucleotides 226100000 to 231000000 from chromosome 2,
   - Region 13b comprises nucleotides 55000000 to 56500000 from chromosome 20,
   - Region 15b comprises nucleotides 179000000 to 182700000 from chromosome 3,
   - Region 19b comprises nucleotides 15200000 to 25200000 from chromosome 6,
   - Region 21b comprising nucleotides 141000000 to 146364022 from chromosome 8 and
   - Region 23b comprises nucleotides 14200000 to 16600000 from chromosome 9
17. The method of aspect 16 wherein the method comprises the determination of the genomic regions 8b and 15b, 8b and 12b, 12b and 23b, 7b and 15b, 7b and 12b, 15b and 23b, 3b and 8b, 8b and 11b, 11b and 23b, 8b and 23b, 3b and 12b, 12b and 15b, 3b and 15b, 3b and 7b, 8b and 19b, 7b and 23b, 3b and 11b, 7b and 11b or 3b and 23b.
18. The method of any one of the preceding aspects, wherein the determination of the presence of a CNA in at least one genomic region is carried out by NGS.
19. The method according to aspect 18 wherein the whole genome sequencing is carried out by shallow whole genome sequencing (shWGS).
20. A method for the treatment of a cancer patient which comprises:
   (i) Predicting the tumor deficiency in the DNA homologous recombination (HR) pathway in the patient suffering from cancer or predicting the response of the cancer to an antitumor treatment by the method as defined in any of aspects 3 to 19 and
   (ii) treating the patient with an antitumor treatment selected from the group consisting of a chemotherapy, targeted therapy or immunotherapy if the patient is determined in step (i) as having a tumor deficiency in the DNA homologous recombination (HR) pathway or predicted in step (i) as being responsive to the tumor treatment.
21. The method according to aspect 20 wherein the antitumor treatment is a treatment that comprises platinum-based drugs or a treatment that comprises a PARPi.
22. A computer system, comprising: one or more processors; a memory; and one or more programs, wherein the one or more programs are stored in the memory and configured to be executed by the one or more processors, the one or more programs including instructions for: performing the method of any one of aspects 1-19.
23. A non-transitory computer-readable storage medium storing one or more programs, the one or more programs comprising instructions, which when executed by one or more processors of an electronic device, cause the electronic device to perform the method of any one of aspects 1-19.

The invention is described below by way of the following examples, which are to be construed as merely illustrative and not limitative of the scope of the invention.

### EXAMPLES

### Materials and Methods

### Sample Selection

A model was trained with a cohort of 100 FFPE ovarian and 100 FFPE breast primary tumor samples with tumor cellularity equal or above 50%.

HRD calculation method described by Marquard et al (HRD-LOH score) was validated using a total of 24 ovarian and 6 breast FFPE tumor samples which HRD scores were previously determined using the FDA-approved HRD test, Myriad MyChoice CDx.

FFPE-paired plasma samples from 6 breast cancer patients were selected to study the correlation of the CNA profile between tissue and plasma samples and therefore the feasibility of our tool for HRD calculation in plasma samples.

### Hybrid capture NGS

FFPE samples were analyzed with a custom hybrid capture-based NGS panel (VHIO-300) that profiles the coding sequences of >450 genes, obtains genome-wide CNA profiles and allows B-allele fraction (BAF) calculation by genotyping 10⁵ single nucleotide polymorphisms (SNPs) distributed throughout the genome. Only samples with tumor cellularity equal or above 50% were included in the training cohort.

Sequencing reads were aligned (BWA v0.7.17, Samtools v1.9) against the hg19 reference genome, base recalibrated, indel realigned (GATK v3.7.0) and variant called (VarScan2 v2.4.3, Mutect2).

The limit of detection of the technique is 5% MAF for SNVs and 10% MAF for INDELs. Frequent SNPs in the population were filtered based on the gnomAD database (allele frequency _ 0.0001). Classification of identified variants was performed using publicly available databases (COSMIC, cBioPortal, ClinVar, VarSome, OncoKB).

Copy number alterations (CNA) were calculated with CNVkit (v0.9.6.dev0) using an in-house 2N pool as normal sample (Talevich, E., et al., 1016, PLoS Comput. Biol. 12, 1-18).

### Shallow Whole Genome Sequencing

Plasma samples were analyzed for shallow whole genome sequencing (shWGS) and the CNA profile was assessed using ichorCNA (v0.2).0.

### HRD-LOH score calculation and validation:

The BAF and CNA information obtained from the analysis of the FFPE samples was used for calculating the HRD score (HRD-LOH Score) using the method described in Marquard et al. (Biomark. Res. 3, 1-10 (2015)) based on 3 genomic scars, LOH, LST and TAI.

### HRD validation method and cut-off determination:

To validate that the HRD-LOH (Marquard) test can be used as a reference test for the HRD status in ovarian and breast cancer, we compared the HRD-LOH and Myriad HRD score in 30 FFPE samples (24 ovarian and 6 breast cancer samples). As a result, a strong Pearson correlation of 0,877 (p-value: 4.557e-11) was obtained (Figure 1).

The line of best fit equation was calculated for the Myriad HRD and HRD-LOH correlation to determine the cut-off for positive HRD status for HRD-LOH in ovarian and breast tumor samples.

The line of best fit equation was also calculated for the HRD-LOH and CNA-HRD correlation and used to establish a cut-off for positive CNA-HRD status in ovarian and breast tumor samples.

### CNA-HRD score calculation for ovarian and breast tumor samples:

CNA segmented data from the 100 FFPE ovarian training cohort was resegmented into delimited genomic regions to allow sample-to-sample comparisons. Length-relative means were computed for each window by considering amplitude values from those segments included in each specific window. Cutoffs for CN Gains and losses were set to log2 values above 0.17 and below -0.193, respectively, that correspond to 3 and 1 copies in samples with 50% tumor cellularity (Figure 3)

In order to identify altered regions with the best correlation with HRD-LOH, 1000 random subsets of the initial 100 samples (each subset containing 75 samples) were generated. In each dataset, a score (CNA-HRD score) was computed that includes all the regions and calculated the positive correlation of each region with the HRD-LOH score. After performing backwards elimination of the regions for each subset as described in Figure 2, the regions that achieved the best correlation during any step of the process were selected (Figure 2).

To compute the CNA-HRD score for ovarian samples, a number of regions were selected as the most stable and repetitive regions obtained by this model training. From these most robust regions a subset was selected based on i) the mean log2 value per region, ii) the number of times that region was altered and iii) the alteration difference between HRD-LOH positive and HRD-LOH negative samples (Figure 4).

The same methodology was applied the breast tumor cohort.

### Example 1: Computation of the CNA-HRD score from tumor samples

After applying the selection model developed by the inventors on the ovarian and breast primary tumor cohorts, 10 CNA regions may serve as effective biomarker to the CNA-HRD score for ovarian and breast cancer (Table 1).

Regions 2, 6, 7, 14, 15, 17, 18, 24 and 25 and regions 3, 7, 8, 11, 12, 13, 15, 19, 21 and 23 were selected to generate a CNA-HRD score for primary ovarian and breast cancer, respectively.

**Table 1. Regions selected based on their stability and repetition (model training), on the log2 value per region, the number of times that region was altered and the alteration difference between HRD-LOH positive and HRD-LOH negative samples.**

| CNA-HRD OVARY | | | |
|---|---|---|---|
| region | chromosome | start | end |
| 17o | chr3 | 153000000 | 155000000 |
| 18o | chr3 | 175700000 | 179000000 |
| 2o | chr11 | 102100000 | 102900000 |
| 3o | chr11 | 121200000 | 127800000 |
| 6o | chr13 | 62300000 | 65700000 |
| 7o | chr13 | 79000000 | 87700000 |
| 14o | chr18 | 66800000 | 68700000 |
| 15ο | chr3 | 8700000 | 11800000 |
| 24o | chr6 | 13400000 | 15200000 |
| 25o | chr6 | 1000000 | 2300000 |

| CNA-HRD BREAST | | | |
|---|---|---|---|
| region | chromosome | start | end |
| 3b | chr12 | 5400000 | 7000000 |
| 7b | chr18 | 25000000 | 32700000 |
| 8b | chr18 | 59000000 | 73100000 |
| 11b | chr19 | 38700000 | 43400000 |
| 12b | chr2 | 226100000 | 231000000 |
| 13b | chr20 | 55000000 | 56500000 |
| 15b | chr3 | 179000000 | 182700000 |
| 19b | chr6 | 15200000 | 25200000 |
| 21b | chr8 | 141000000 | 146000000 |
| 23b | chr9 | 14200000 | 16600000 |

As shown in Figure 5, both scores (CNA-HRD Ovary and CNA-HRD Breast) have a strong Pearson correlation of 0,805 (pval<2.2e-16) and 0.817 (pval<2.2e-16) with HRD-LOH, respectively. BRCA-LOH mutations are also displayed in the plot.

If checked individually, for ovarian cancer, only 2 out of the 10 regions obtained a correlation (R) ≥ 0.50 with HRD-LOH (reg17 and reg18) indicating that these 2 regions could be key in the CNA-HRD score estimation. As for the rest, random combination between any two of them resulted in a correlation with HRD-LOH ≥ 0.50. Some examples of randomly combined regions and the correlation with the HRD-LOH score are shown in Table 2.

The same was analyzed for breast cancer. It was observed that random combinations of 2 regions were enough to compute a CNA-HRD score that correlates with HRD-LOH (Table 2).

**Table 2. Best examples of correlation between HRD-LOH and CNA-HRD Score generated from individual or randomly combined regions.**

| | OVARY | |
|---|---|---|
| | Region/s | R |
| Individually | reg17o | 0.57 |
| | reg18o | 0.66 |
| Combination between 2 of them | reg_6o&25o | 0.52 |
| | reg_3o&14o | 0.54 |
| | reg_2o&3o | 0.54 |
| | reg_2o&7o | 0.55 |
| | reg_15o&24o | 0.55 |
| | reg_14o&24o | 0.55 |
| | reg_2&o24o | 0.55 |
| | reg_15o&25o | 0.55 |
| | reg_2o&15o | 0.55 |
| | reg_3o&7o | 0.55 |
| | reg_6o&14o | 0.56 |
| | reg_3o&15o | 0.56 |
| | reg_2o&6o | 0.57 |
| | reg_3o&24o | 0.57 |
| | reg_2o&25o | 0.57 |
| | reg_3o&25o | 0.58 |
| | reg_3o&6o | 0.59 |
| | reg_14o&25o | 0.59 |
| | reg_2o&14o | 0.61 |

| | BREAST | |
|---|---|---|
| | Regions | R |
| Combination between 2 of them | reg_8b&1b5 | 0.61 |
| | reg_8b&12b | 0.58 |
| | reg_12b&23b | 0.58 |
| | reg_7b&15b | 0.56 |
| | reg_7b&12b | 0.56 |
| | reg_15b&23b | 0.55 |
| | reg_3b&8b | 0.55 |
| | reg_8b&11b | 0.55 |
| | reg_11b&23b | 0.55 |
| | reg_8b&23b | 0.54 |
| | reg_3b&12b | 0.54 |
| | reg_12b&15b | 0.53 |
| | reg_3b&15b | 0.52 |
| | reg_3b&7b | 0.52 |
| | reg_8b&19b | 0.52 |
| | reg_7b&23b | 0.51 |
| | reg_3b&11b | 0.51 |
| | reg_7b&11b | 0.51 |
| | reg_3b&23b | 0.50 |

As explained in methods, the line of best fit equation when comparing HRD score by Marquard and Myriad was y = 0.8091x + 22.178. Based on the stablished cut off of HRD ≥ 42 for Myriad, a cut-off for positive HRD status was stablished for HRD-LOH (Marquard) in ovarian and breast tumor samples when HRD score ≥ 56. (Figure 1)

The line of best fit equation when comparing both HRD-LOH and CNA-HRD was y = 0.8382x + 2.9889 and y = 0.8485x - 3.9414 for ovarian and tumor samples, respectively. With a cut-off of HRD ≥ 56 for HRD-LOH, CNA-HRD cut-off ≥ 50 and ≥ 44 were stablished for positive HRD status in primary ovarian and breast cancer, respectively (Figure 5).

### Example 2: Determination of CNA-HRD score in biofluid samples

In order to assess the feasibility of CNA-HRD score in liquid biopsy, the authors of the present invention analyzed 6 plasma samples from the breast cancer cohort and compare them with their tissue partner. CNA patterns were extracted from each sample (plasma and tissue) and the log2 calculated for each of the 10 regions that compose the breast CNA-HRD score. The correlation between the tissue and plasma log2 values and the correlation coefficient (R) for each patient are shown in Figure 6 and summarized in Figure 7 where the time extraction difference between the tissue and liquid biopsy (Dt extraction) is indicated.

A high correlation (R from 0.706 to 0.931) is observed for samples where the time extraction difference was no longer than 12 days. Moreover, correlations above 0.895 were found in samples where time extraction difference was almost a year difference. These results demonstrate, not only the feasibility of CNA-HRD score as potent surrogate biomarkers to determine HR status in liquid biopsy, but also the correlation of this biomarker over the time.

## Claims

1. An *in vitro* method for determining the DNA homologus recombination (HR) status of a tumor, comprising determination in a biological sample of said tumor the presence of a copy number alteration (CNA) in at least one genomic region, wherein the genomic region is selected from the group consisting of the genomic regions as shown in Table 1 wherein the presence of said at least one CNA in at least one genomic region is correlated with a HR-deficient status.

2. The method according to claim 1 wherein the tumor sample is a sample from a primary tumor or a metastasis.

3. An in vitro method for predicting tumor deficiency in the DNA homologous recombination (HR) pathway in a patient suffering from cancer, comprising determining in the genetic material of a sample from said patient the presence of CNA in at least one genomic region selected from the group consisting of the genomic regions as shown in Table 1, wherein the presence of said at least one CNA indicates that the tumor in the patient has a HR-deficient status.

4. The method according to claim 3 wherein the sample is either a tumor sample or a biofluid sample.

5. The method according to claim 4 wherein the tumor sample is a sample from a primary tumor or a metastasis.

6. The method according to claim 4 wherein the biofluid is blood or a derivative thereof.

7. The method according to any of claims 3 to 6 wherein the cancer patient suffers from, but not limited to, ovarian, breast cancer, pancreatic cancer, prostate cancer or sarcoma.

8. The method according to claim 7 wherein the cancer patient suffers from ovarian or breast cancer

9. An *in vitro* method for predicting the response of a cancer patient to an antitumor treatment, said method comprising determining in the genetic material of a sample from said patient the presence of CNA in at least one genomic region selected from the group consisting of the genomic regions as shown in Table 1, wherein the presence of said at least one CNA indicates that the cancer is responsive to an antitumor treatment selected from the group consisting of a chemotherapy, targeted therapy or immunotherapy.

10. The method of claim 10 wherein the antitumor treatment is a treatment that comprises platinum-based drugs or a treatment that comprises a PARPi.

11. The method according to any preceding claim wherein the method comprises the determination of the CNA in at least two genomic regions.

12. The method according to any preceding claim wherein the cancer is ovarian cancer and wherein the at least one genomic region is selected from the group consisting of regions 2o, 3o, 6o, 7o, 14o, 15o, 17o, 18o and 25o wherein:
- Region 2 comprises nucleotides 102100000 to 102900000 from chromosome 11
- Region 3 comprises nucleotides 121200000 to 127800000 from chromosome 11
- Region 6 comprises nucleotides 62300000 to 65700000 from chromosome 13
- Region 7 comprises nucleotides 79000000 to 87700000 from chromosome 13
- Region 14 comprises nucleotides 66800000 to 68700000 from chromosome 18,
- Region 15 comprises nucleotides 8700000 to 11800000 from chromosome 3,
- Region 17 comprises nucleotides 153000000 to 155000000 from chromosome 3,
- Region 18 comprises nucleotides 175700000 to179000000 from chromosome 3,
- Region 24 comprises nucleotides 13400000 to 15200000 from chromosome 6 and,
- Region 25 comprises nucleotides 1000000 to 2300000 from chromosome 6

13. The method according to claim 14 wherein the method comprises the determination of the genomic regions 6o and 25o, 3o and 14o, 2o and 3o, 2o and 7o, 15o and 24o, 14o and 24o, 2o and 24o, 15o and 25o, 2o and 15o, 3o and 7o, 6o and 14o, 3o and 15o, 2o and 6ο, 3o and 24o, 2o and 25o, 3o and 25o, 3o and 6ο, 14o and 25o or 2o and 14o.

14. The method according to any preceding claim wherein the cancer is breast cancer and wherein the at least one genomic region is selected from the group consisting of the 3b, 7b, 8b, 11b, 12b, 13b, 15b, 19b, 21b and 23b regions wherein:
- Region 3b comprises nucleotides 5400000 to 7000000 from chromosome 12,
- Region 7b comprises nucleotides 25000000 from 32700000 in chromosome 18,
- Region 8b comprises nucleotides 59000000 to 73100000 from chromosome 18,
- Region 11b comprises nucleotides 38700000 to 43400000 from chromosome 19,
- Region 12b comprises nucleotides 226100000 to 231000000 from chromosome 2,
- Region 13b comprises nucleotides 55000000 to 56500000 from chromosome 20,
- Region 15b comprises nucleotides 179000000 to 182700000 from chromosome 3,
- Region 19b comprises nucleotides 15200000 to 25200000 from chromosome 6,
- Region 21b comprising nucleotides 141000000 to 146364022 from chromosome 8 and
- Region 23b comprises nucleotides 14200000 to 16600000 from chromosome 9

15. The method of claim 16 wherein the method comprises the determination of the genomic regions 8b and 15b, 8b and 12b, 12b and 23b, 7b and 15b, 7b and 12b, 15b and 23b, 3b and 8b, 8b and 11b, 11b and 23b, 8b and 23b, 3b and 12b, 12b and 15b, 3b and 15b, 3b and 7b, 8b and 19b, 7b and 23b, 3b and 11b, 7b and 11b or 3b and 23b.
